(19) Europäisches Patentamt
European Patent Office
Office européen des brevets

(11) Veröffentlichungsnummer: **0 087 666**
**B1**

(12) # EUROPÄISCHE PATENTSCHRIFT

(45) Veröffentlichungstag der Patentschrift: **02.05.85**

(51) Int. Cl.⁴: **C 07 C 102/10,** C 07 C 103/133, B 01 J 23/86 // C07C131/00

(21) Anmeldenummer: **83101387.5**

(22) Anmeldetag: **14.02.83**

(54) **Verfahren zur Herstellung von Methacrylamid.**

(30) Priorität: **19.02.82 DE 3205946**

(43) Veröffentlichungstag der Anmeldung: **07.09.83 Patentblatt 83/36**

(45) Bekanntmachung des Hinweises auf die Patenterteilung: **02.05.85 Patentblatt 85/18**

(84) Benannte Vertragsstaaten: **BE CH DE FR GB LI**

(56) Entgegenhaltungen:
**DE - A - 2 343 292**
**DE - B - 1 044 795**

(73) Patentinhaber: **BASF Aktiengesellschaft, Carl-Bosch-Strasse 38, D-6700 Ludwigshafen (DE)**

(72) Erfinder: **Platz, Rolf, Dr., Hansastrasse 5, D-6800 Mannheim (DE)**
Erfinder: **Gath, Rudolph Hans, Dr., Ellenburger Weg 41, D-6800 Mannheim (DE)**
Erfinder: **Petersen, Harro, Dr., Kalmitstrasse 23, D-6710 Frankenthal (DE)**

**EP 0 087 666 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur Herstellung von Methacrylamid durch Umsetzung von Methacroleinoxim in Gegenwart von inerten Lösungsmitteln und eines Kupfer/Chrom-Katalysators mit einem Verhältnis von 0,01 bis 0,5 Chrom zu G-Atom Kupfer.

Es ist aus der DE-PS Nr. 1044795 bekannt, dass man Carbonsäureamide durch Isomerisierung von Aldoximen mittels der Behandlung mit wässerigen Lösungen oder Suspensionen von Verbindungen von Metallen der VIII. Hauptgruppe, I. oder II. Nebengruppe des Periodischen Systems erhält. Als Ausgangsstoffe werden in den Beispielen im wesentlichen gesättigte aliphatische Aldoxime wie Butyraldoxim bzw. das aromatische Benzaldoxim, aufgeführt. Als einziges ungesättigtes Aldoxim wird Zimtantialdoxim durch ein Beispiel mit einem Nickelkatalysator gezeigt; unter den 18 Beispielen zeigt dieses ungesättigte Oxim die zweitschlechteste Ausbeute von 70%. Acroleinoxim wird lediglich in einer Aufzählung erwähnt. Methacroleinoxim wird nicht geoffenbart. Ein wesentliches Merkmal des Verfahrens ist das wässerige Reaktionsmedium. Nur in einem der 18 Beispiele, bei der Umsetzung von n-Butyraldoxim, wird eine Kupferverbindung (Acetat) beschrieben.

Es wurde nun gefunden, dass man Methacrylamid durch Isomerisierung von Aldoximen in Gegenwart von Kupferkatalysatoren und einem Lösungsmittel vorteilhaft herstellt, wenn man Methacroleinoxim der Formel

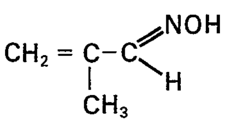

(I)

in Gegenwart von Katalysatoren, die Kupfer und Chrom in einem Verhältnis von 0,01 bis 0,5 G-Atom Chrom zu 1 G-Atom Kupfer enthalten, und in Gegenwart von unter den Reaktionsbedingungen inerten, organischen Lösungsmitteln umsetzt.

Die Umsetzung wird durch die folgenden Formeln wiedergegeben

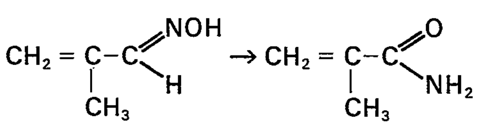

Im Hinblick auf das bekannte Verfahren liefert das Verfahren nach der Erfindung auf einfachem und wirtschaftlichem Wege Methacrylamid in besserer (Beispiel und Vergleichsbeispiel) Ausbeute und Reinheit. Die Verwendung des gerade im grosstechnischen Massstab nachteiligen, toxischen Nickels wird vermieden. Alle diese vorteilhaften Ergebnisse sind im Hinblick auf den Stand der Technik überraschend. Man hätte erwarten müssen, dass Nickel- und nicht Kupferkatalysatoren für ungesättigte Aldoxime besonders geeignet sind. Auch ist es überraschend, dass gerade die Kombination von Kupfer und Chrom im Katalysator für die erfindungsgemässe Umsetzung vorteilhaft ist. Ganz im Gegensatz zur Lehre der deutschen Patentschrift konnte nicht erwartet werden, dass organische Lösungsmittel anstelle wässeriger Suspensionen für die Methacrylamidherstellung verwendet werden müssen. Eine technisch wirtschaftliche Synthese von Methacrylamid ist so möglich, da man das Methacroleinoxim, das durch Umsetzung von Propionaldehyd mit Formaldehyd und anschliessender Wasserabspaltung und Oximierung mit Hydroxylamin erhältlich ist, in guten Ausbeuten zum Amid umlagern kann. Nebenreaktionen, z.B.

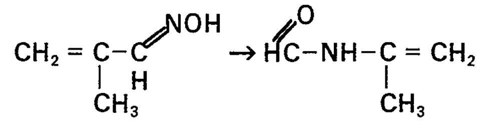

oder Polymerisationen an der Doppelbindung des Methacroleinoxims bzw. des Methacrylamids treten nicht in deutlichem Masse auf.

Die Umsetzung wird im allgemeinen bei einer Temperatur von 10 bis 100, vorzugsweise von 40 bis 90, insbesondere von 50 bis 70° C, drucklos oder unter Druck, vorteilhaft dem sich einstellenden Eigendruck des Reaktionsgemisches, kontinuierlich oder diskontinuierlich durchgeführt. Man setzt in Gegenwart von unter den Reaktionsbedingungen inerten, organischen Lösungsmitteln um. Als Lösungsmittel kommen z.B. in Frage: aromatische Kohlenwasserstoffe, z.B. Toluol, Ethylbenzol, o-, m-, p-Xylol, Isopropylbenzol, Methylnaphthalin; Halogenkohlenwasserstoffe, insbesondere Chlorkohlenwasserstoffe, z.B. Tetrachlorethylen, 1,1,2,2- oder 1,1,1,2-Tetrachlorethan, Amylchlorid, Cyclohexylchlorid, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, 1,2-Dichlorethan, 1,1-Dichlorethan, n-Propylchlorid, 1,2-cis-Dichlorethylen, n-Butylchlorid, 2-, 3- und iso-Butylchlorid, Chlorbenzol, Fluorbenzol, o-, p- und m-Dichlorbenzol, o-, m-, p-Chlortoluol, 1,2,4-Trichlorbenzol; aliphatische oder cycloaliphatische Kohlenwasserstoffe, z.B. Heptan, α-Pinen, Pinan, Nonan, Benzinfraktionen innerhalb eines Siedepunktintervalls von 70 bis 190° C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; und entsprechende Gemische. Zweckmässig verwendet man das Lösungsmittel in einer Menge von 40 bis 10 000, vorzugsweise von 50 bis 1000 Gew.-%, bezogen auf Ausgangsstoff II. Bevorzugt sind Toluol, Xylol, Cyclohexan, Heptan. Als Kupfer/Chrom-Katalysatoren kommen a) Kupfer und/oder Kupferverbindungen, b) Chromverbindungen, zweckmässig in Gestalt von Trägerkatalysatoren, in Betracht. Der Katalysator kann eine oder mehrere Kupferverbindungen und eine oder mehrere Chromverbindungen oder gemeinsam Kupfer und eine oder mehrere Kupferverbindungen und Chromverbindungen enthalten; bevorzugt ist die Kombination von einer Kupferverbindung und einer Chromverbindung. Die Kupferverbindungen

können solche des ein- oder zweiwertigen Kupfers sein. Es kommen als Kupferanteil und Chromanteil der Katalysatoren in Frage; atomares Kupfer, Raney-Kupfer; Kupfer(II)acetat, Kupfer(II)arsenat, Kupfer(II)arsenit, Kupfer(II)borat, Kupfer(II)bromid, Kupfer(II)oxybromid, Kupfer(II)chlorid, Kupfer(II)formiat, Kupfer(II)hydroxid, Kupfer(II)-nitrat, Kupfer(II)oxid, Kupfer(II)phosphat, Kupfer(II)rhodanid, Kupfer(II)sulfat, Kupfer(II)sulfit, Kupfer(II)sulfid, Kupfer(II)tartrat, Kupfer(II)fluorid, Kupfer(II)nitrit, Kupfer(II)cyanid, Kupfer(II)-molybdat; Kupfer(I)bromid, Kupfer(I)chlorid, Kupfer(I)hydroxid, Kupfer(I)jodid, Kupfer(I)oxid, Kupfer(I)rhodanid, Kupfer(I)sulfid, Kupfer(I)sulfit, Kupfer(I)sulfat; Chromverbindungen mit vorgenannten Anionen. Im allgemeinen verwendet man den Katalysator (ohne Träger) in einer Menge von 0,3 bis 20, bevorzugt von 0,5 bis 15, insbesondere 1 bis 10 Gew.-% Gesamtkupfer + Gesamtchrom, bezogen auf Ausgangsstoff I; Gesamtkupfer (entsprechend auch Gesamtchrom) wird als Gesamtsumme von freiem und in irgendeiner Form, z.B. Salzform, Additionsverbindung, Komplexverbindung, Legierung, gebundenem Kupfer (oder Chrom) im Katalysator definiert. Bevorzugt sind CuO und $Cr_2O_3$ bzw. solche Verbindungen, die während der Reaktion diese Oxide bilden.

Als Träger kommen in Frage Kieselsäureverbindungen wie Silikate, z.B. Natriumaluminiumsilikat, Calciumaluminiumsilikat, Bleicherden, Fullererde, Tone, Kaolin, Meerschaum, Allophane, Zeolithe, Montmorillonit, Bimsstein, Floridaerde, Quarz, Asbest, Mullit, Bentonit; gefällte Kieselsäure, Kieselgel, Kieselgur, Siliciumcarbib; α- und γ-Aluminiumoxide und -hydroxide, z.B. Korund, γ-Tonerde, Hydrargillit, Böhmit, Bauxit; Aluminiumsilikate, z.B. Andalusit; Titandioxid, Zirkondioxid, Zinndioxid, Magnesit, Aktivkohle, Zinkoxid; Erdalkalisulfate oder Erdalkaliphosphate, z.B. die Calcium- oder Bariumsalze; Metalloxide, z.B. Aluminiumoxid, Zinkoxid, Calciumoxid; oder entsprechende Gemische vorgenannter Trägermaterialien. Die Herstellung der Trägerkatalysatoren wird nach den üblichen Verfahren, z.B. durch Auftragen der Kupfer- und Chromverbindung bzw. des Kupfers auf den Träger, Trocknen und Calcinieren, beispielsweise zwischen 400 und 1200° C in reduzierender, oxidierender oder inerter Atmosphäre, durchgeführt. Der Träger kann auch in seiner gewünschten geometrischen Form mit einer Lösung der Kupfer- und Chromverbindung, z.B. einer wässerigen Lösung von Kupfersulfat, getränkt und getrocknet bzw. das Kupfer und $Cr_2O_3$ durch Erhitzen ausgefällt werden. Ebenfalls kann man das Trägermaterial mit Kupfer oder der Kupfer- und Chromverbindung und Wasser verkneten, in die gewünschte Form bringen, trocknen und bei einer Temperatur von 400 bis 1200° C calcinieren. Die Teilchengrösse der Katalysatoren auf dem Träger beträgt vorzugsweise von 0,05 bis 7 mm. Die Form kann beliebig, z.B. in Pillen-, Zylinder- oder Strangform, kugelförmig oder körnig, gewählt werden. Bevorzugt sind bei dem Katalysator auf Träger Porenvolumina von 0,05 bis 1 ml/g, spezifische Oberflächen von 1 bis 300 m²/g, mittlere Porenporösitat von 0,32 bis 0,64 cm³/g und Schüttgewichte von 0,4 bis 2,1 g/ml. Vorteilhaft enthalten die Trägerkatalysatoren 20 bis 40, insbesondere 30 bis 35 Gew.-% Gesamtkupfer + Gesamtchrom, bezogen auf den Träger.

Als Nebenkomponenten sind Erdalkaliverbindungen (insbesondere Barium-, Strontium-, Magnesiumverbindungen) und Zinkverbindungen, z.B. in Form ihrer Oxide, Carbonate, Hydroxide, Bicarbonate und Hydroxidcarbonate, anwesend. Bevorzugt sind Katalysatoren der Kombination

$$CuO \cdot Cr_2O_3 \cdot ZnO \cdot BaO$$

auf Träger, bevorzugt Silikate wie Magnesiumsilikat. Man verwendet 0,01 bis 0,5, vorzugsweise 0,01 bis 0,1 G-Atom Chrom je Grammatom Kupfer, gleichgültig in welchen Verbindungen sie vorliegen. Es kommen zur Anwendung Kombinationen von 10 bis 50, insbesondere 20 bis 40 Gew.-% Kupferverbindungen (insbesondere Kupfer(II)-oxid), von 0,1 bis 10 insbesondere 0,1 bis 1 Gew.-% Chromverbindungen (insbesondere $Cr_2O_3$), 0,1 bis 5, inbesondere 0,1 bis 1 Gew.-% Zinkverbindungen (insbesondere ZnO), 0,1 bis 5, insbesondere 0,1 bis 1 Gew.-% Erdalkaliverbindungen (insbesondere BaO); die Restmenge auf 100% ist Träger. Ein besonders bevorzugter Katalysator hat die Zusammensetzung 36,5% CuO, 0,6% $Cr_2O_3$, 0,4% ZnO, 1% BaO; 61,5% Träger (insbesondere Magnesiumsilikat).

Die Katalysatoren auf dem Träger können auch in Splitt- oder Kugelform in der Wirbelschicht eingesetzt werden, wobei zweckmässig Katalysatorteilchen mit Korngrössen von 0,005 bis 3,0, insbesondere von 0,1 bis 1,0 mm, verwendet werden. Die Schichthöhe des Katalysatorbettes wird zweckmässig so gewählt, dass sich Verweilzeiten der Ausgangsstoffe II in der Katalysatorschicht von 0,01 bis 20, vorzugweise von 1 bis 10 s, ergeben. Bezüglich der Herstellung der Katalysatoren wird auf Houben-Weyl, „Methoden der Organischen Chemie", Bd. 4/2, S. 142 ff. und „ Ullmann's Encyclopädie der technischen Chemie" (3. Aufl.), Bd. 9, S. 271 ff. verwiesen.

Die Reaktion kann wie folgt durchgeführt werden: Ein Gemisch von Ausgangsstoff I, Katalysator und Lösungsmittel, wird während 1 bis 6 h bei der Reaktionstemperatur gehalten. Dann wird der Endstoff aus dem Gemisch in üblicher Weise, z.B. durch Filtration nach Kristallisation, abgetrennt.

Das nach dem Verfahren herstellbare Methacrylamid ist ein wertvoller Ausgangsstoff für die Herstellung von Pflanzenschutzmitteln und Kunststoffen. Bezüglich der Verwendung wird auf die vorgenannte Veröffentlichung und Ullmann's Werk (loc. cit., 4. Aufl., Bd. 16, S. 612) verwiesen.

*Beispiel*

*Umlagerung von Methacroleinoxim in Toluol*

50 g Methacroleinoxim wurden in 200 ml Toluol gelöst. Nach Zusatz von 10 g Katalysator mit 36,5% CuO, 0,6% $Cr_2O_3$, 0,4% ZnO, 1% BaO; 61,5% Träger (Magnesiumsilikat) wurde das Gemisch 6 h unter Rückfluss (111° C) umgesetzt.

Nach dem Abkühlen wurden 18 g Endstoff abgesaugt. Das Filtrat wurde eingeengt. Man erhielt 36 g Methacrylamid (72% der Theorie) vom Fp. 109° C.

*Vergleichsbeispiel*

*Umlagerung von Methacroleinoxim im Druckautoklaven bei 2,5 bar in Wasser*

Analog dem vorstehenden Beispiel wurde die Umsetzung anstelle von Toluol in 800 ml Wasser mit pulverisiertem Katalysator bei 100° C und 2,5 bar 6 h durchgeführt. Man erhielt 17,5 g Methacrylamid (35% der Therorie) vom Fp. 109° C.

**Patentanspruch**

Verfahren zur Herstellung von Methacrylamid durch Isomerisierung von Aldoximen in Gegewart von Kupferkatalysatoren und einem Lösungsmittel, dadurch gekennzeichnet, dass man Methacroleinoxim der Formel

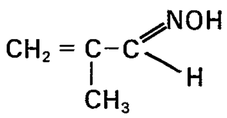

(I)

in Gegenwart von Trägerkatalysatoren, die Kupfer und Chrom in einem Verhältnis von 0,01 bis 0,5 G-Atom Chrom zu 1 G-Atom Kupfer enthalten, mit einem Verhältnis von 10 bis 50 Gew.-% Kupferverbindungen, von 0,1 bis 10 Gew.-% Chromverbindungen, 0,1 bis 5 Gew.-% Zinkverbindungen und 0,1 bis 5 Gew.-% Erdalkaliverbindungen im Trägerkatalysator in Gegenwart von unter den Reaktionsbedingungen inerten, organischen Lösungsmitteln umsetzt.

**Claim**

A process for the preparation of methacrylamide by isomerization of an aldoxime in the presence of a copper catalyst and a solvent, wherein methacrolein oxime of the formula

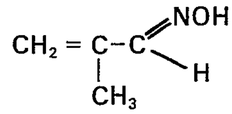

(I)

is reacted in the presence of a supported catalyst which contains chromium and copper in a gramme-atom ratio of 0.01 to 0.5: 1, and from 10 to 50% by weight of copper compounds, from 0.1 to 10% by weight of chromium compounds, from 0.1 to 5% by weight of zinc compounds and from 0.1 to 5% by weight of alkaline earth metal compounds, and in the presence of an organic solvent which is inert under the reaction conditions.

**Revendication**

Procédé de préparation de l'amide méthacrylique par isomérisation d'aldoximes dans un solvant en présence d'un catalyseur au cuivre, caractérisé en ce que l'on fait réagir l'oxime de la méthacroléine de la formule

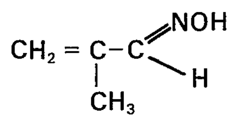

(I)

dans des solvants organiques inertes dans les conditions opératoires en présence de catalyseurs sur support, qui contiennent du cuivre et du chrome dans un rapport de 0,01 à 0,5 atome-gramme de chrome par atome-gramme de cuivre et dans lesquels les proportions respectives sont de 10 à 50% en poids de dérivés du cuivre, 0,1 à 10% en poids de dérivés du chrome, de 0,1 à 5% en poids de dérivés du zinc et de 0,1 à 5% en poids de dérivés de métaux alcalino-terreux.